(19) Europäisches Patentamt
European Patent Office
Office européen des brevets

(11) Veröffentlichungsnummer: **0 306 844 A2**

# (12) EUROPÄISCHE PATENTANMELDUNG

(21) Anmeldenummer: **88114312.7**

(51) Int. Cl.[4]: **A61K 31/52**

(22) Anmeldetag: **02.09.88**

(30) Priorität: **11.09.87 DE 3730541**

(43) Veröffentlichungstag der Anmeldung:
**15.03.89 Patentblatt 89/11**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Winkelmann, Erhardt, Dr.**
**Brunhildenweg 5**
**D-6233 Kelkheim (Taunus)(DE)**
Erfinder: **Winkler, Irvin, D.**
**In den Eichen 40**
**D-6237 Liederbach(DE)**
Erfinder: **Meichsner, Christoph, Dr.**
**Bienerstrasse 30**
**D-6238 Hofheim am Taunus(DE)**

## (54) Verwendung bestimmter 6-Mercaptopurine zur Herstellung eines Arzneimittels sowie Verfahren zur Herstellung des Arzneimittels.

(57) Verwendung bestimmter 6-Mercaptopurine zur Herstellung eines Arzneimittels zur Behandlung von durch Retroviren -insbesondere durch HIV - verursachten Krankheiten.

EP 0 306 844 A2

Xerox Copy Centre

## Verwendung bestimmter 6-Mercaptopurine zur Herstellung eines Arzneimittels sowie Verfahren zur Herstellung des Arzneimittels

Es ist bekannt, daß verschiedene Purinderivate wertvolle chemotherapeutische Eigenschaften besitzen. So entfaltet z.B. das Handelsprodukt 6-Mercaptopurin (= 6-Purinthiol) eine in vivo Wirkung gegen bestimmte Krebsarten, insbesondere gegen Leukämie; 6-Mercaptopurin ist die Verbindung der Formel

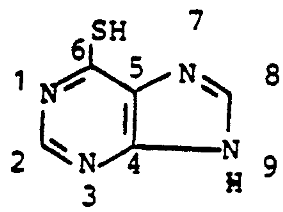

In der DE-A-36 27 024 werden verschiedene weitere Purinderivate - darunter ebenfalls solche mit einer Mercaptogruppe in 6-Stellung - beschrieben und auch beansprucht, welche sich zur Bekämpfung von durch Viren -und zwar sowohl durch DNS- als auch durch RNS-Viren -verursachten Krankheiten sowie von Autoimmun-Krankheiten und zur Behandlung von Krebs eignen sollen. DNS-Viren sind z.B. die Pocken- und Herpesviren, RNS-Viren z.B. die Polioviren; die derzeit wohl meistdiskutierten RNS-Viren sind die HIV (= human immuno deficiency-viruses).

Die in der vorerwähnten DE-A beanspruchten Purinderivate besitzen die Formel

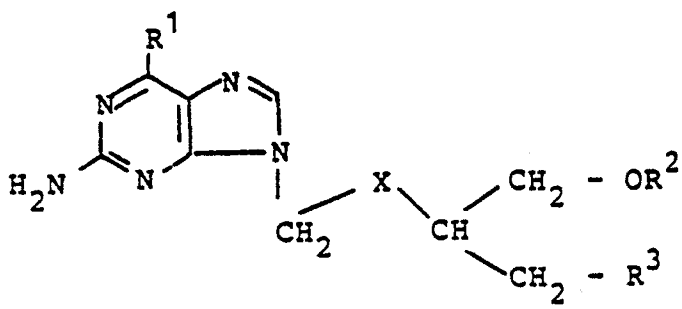

worin $R^1$ = H, Halogen, OH, SH, Azido, $NH_2$ oder Z-$R^5$,
Z = O, S, SO, $SO_2$ oder NH,
$R^5$ = acyclischer $C_1$-$C_6$-Kohlenwasserstoffrest, welcher
a) unsubstituiert ist oder
b) durch wenigstens eines der Merkmale modifiziert ist, daß er eine Alkoxygruppe mit 1 - 3 C-Atomen oder eine Doppelbindung enthält oder mindestens teilweise fluoriert ist, oder
$C_3$-$C_6$-Cycloalkyl oder
Phenylalkyl, das direkt oder über eine $C_1$-$C_3$-Alkylengruppe an Z gebunden ist, oder
Phenyl, wobei der Phenylring jeweils durch Halogen, $CF_3$, Alkoxy oder Alkylthio mit jeweils 1 - 3 C-Atomen substituiert sein kann,
$R^2$ = H oder von der Bedeutung wie $R^5$, oder $COR^6$
$R^6$ = $C_1$-$C_6$-Alkyl, Benzyl oder Phenyl,
$R^3$ = Halogen, Azido, $NH_2$ oder Y-$R^4$,
$R^4$ = gleiche Bedeutung wie $R^5$, und
X, Y = O, S, SO, $SO_2$ und X und Y gleich oder verschieden sein können,
wobei solche Verbindungen ausgenommen sind, in denen nebeneinander
$R^1$ = OH oder SH,
$R^2$ = H,
$R^3$ = Y-$R^4$
X = O oder S,
Y = O und

$R^4$ = $C_1$-$C_4$-Alkyl außer i-$C_3H_7$ und sec.-$C_4H_9$,
bzw. in denen nebeneinander
$R^1$ = OH oder $NH_2$,
$R^2$ = Benzyl,
$R^3$ = Y-$R^4$ mit
Y = O oder
$R^4$ = Benzyl, und
X = O.

In Weiterverfolgung der Arbeiten gemäß der vorerwähnten DE-A wurde gefunden, daß eine Reihe weiterer bekannter 6-Mercaptopurine, von denen auch bereits andere medizinische Indikationen bekannt sind, eine gute Wirksamkeit gegen Krankheiten besitzen, welche speziell durch Retroviren -insbesondere durch HIV = verursacht werden; dies stellt eine wertvolle Bereicherung in der schwierigen Chemotherapie derartiger Krankheiten dar. Die hier in Frage kommenden 6-Mercaptopurine besitzen die folgende Formel I

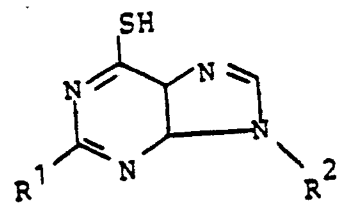

(I)

worin $R^1$ = H, $NH_2$, $NHR^{1'}$ ($R^{1'}$ = subst. Acylrest, vorz. $COCH_3$),

$$R^2 = H,\ -CH_2-X-\underset{R^5}{CH}-CH_2-OR^3,$$

X = O oder S
$R^3$ = H, aliphat. $C_1$-$C_6$-Kohlenwasserstoffrest mit ggf. 1 Doppelbindung, $CH_2$-$C_6H_5$, $COR^4$,
$R^4$ = $C_1$-$C_6$-Alkylrest, $CH_2$-$C_6H_5$, $C_6H_5$,
$R^5$ = H, $CH_2$-Y,
Y = H, Halogen, $OR^6$,
$R^6$ = unabhängig von $R^3$ die gleichen Bedeutungen wie $R^3$, mit Ausnahme der Verbindungen, in denen
a) $R^1$ = $NH_2$ und
$R^5$ (in $R^2$) = $CH_2$-Y mit
Y = Halogen oder $OR^6$, ,
$R^6$ = aliphat. $C_1$-$C_6$-Kohlenwasserstoffrest mit ggf. 1 Doppelbindung oder $CH_2$-$C_6H_5$, sowie
b) $R^1$ = H und

$$R^2 = -CH_2-X-\underset{R^5}{CH}-CH_2OR^3 \text{ mit}$$

X = O oder S,
$R^3$ = H, i-$C_3H_7$, sec-$C_4H_9$, $CH_2$-$C_6H_5$, $COR^4$,
$R^4$ = $CH_3$, $C_2H_5$, $C_6H_5$,
$R^5$ = H, $CH_2OC_3H_7$(i), $CH_2OC_4H_9$(sec.), $CH_2OCOR^4$,
wobei die Verbindungen mit gleichzeitig $R^1$ = $R^3$ = $R^5$ = H nicht ausgenommen sind.

Erfindungsgegenstand ist somit die Verwendung der Verbindungen der Formel I zur Herstellung eines Arzneimittels zur Behandlung von durch Retroviren - insbesondere durch HIV - verursachten Krankheiten.

Die Arzneimittel, welche die Verbindungen enthalten, können enteral (oral), parenteral (intravenös), rektal oder lokal (topisch) angewendet werden. Sie können in Form von Lösungen, Pulvern, Tabletten, Kapseln, einschließlich Mikrokapseln, Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigenzien, Farbstoffe und/oder Puffersubstanzen in Frage.

Als zweckmäßige Dosierung werden etwa 0,1 von 10 mg, vorzugsweise etwa 0,2 bis 8 mg/kg Körpergewicht verabreicht. Die Verabreichung erfolgt zweckmäßig in Dosierungseinheiten, die mindestens die wirksame Tagesmenge der Verbindungen enthalten; d.i. vorteilhaft etwa 30 bis 300 mg, insbesondere etwa 50 bis 250 mg.

Zur Herstellung des Arzneimittels wird eine wirksame Menge mindestens einer Verbindung der Formel I mit üblichen physiologisch verträglichen Zusatzstoffen in eine geeignete Darreichungsform gebracht.

Auch die Verabreichung der Verbindungen der Formel I in Kombination mit z.B. anderen Antivirusmitteln und Immunstimulantien (Interferone, etc.) ist möglich.

Die von Formel I unter a ausgenommenen Verbindungen sind aus der vorerwähnten DE-A - auch für die gleiche Indikation -bekannt;
die unter b ausgenommenen Verbindungen sind in der gleichzeitig eingereichten Anmeldung P 37 30 542.5 (HOE 87/F 264 ) - ebenfalls für die gleiche Indikation -beansprucht.

Einige beispielhafte Verbindungen der Formel I sind:

| Verbindungen der Formel I | $R^1$ | $R^2$ |
|---|---|---|
| 6-Mercaptopurin | H | H |
| 6-Mercapto-9-(2-hydroxyethoxymethyl)-purin | " | $-CH_2-O-CH_2-CH_2OH$ |
| 6-Mercapto-9-(1,3-dibenzyloxypropyl-2-oxymethyl)-purin | " | $-CH_2-O-CH(CH_2OCH_2C_6H_5)_2$ |
| 6-Mercapto-9-(1,3-dihydroxypropyl-2-oxymethyl)-purin | " | $-CH_2-O-CH(CH_2OH)_2$ |
| 2-Amino-6-mercaptopurin | $NH_2$ | H |
| 2-Amino-6-mercapto-9-(2-hydroxyethoxymethyl)-purin | " | $-CH_2-O-CH_2-CH_2OH$ |
| 2-Amino-6-mercapto-9-(2-benzyloxy-ethoxymethyl)-purin | " | $-CH_2-O-CH_2-CH_2-OCH_2C_6H_5$ |
| 2-Amino-6-mercapto-9-(1,3-dihydroxypropyl-2-oxymethyl)-purin | " | $-CH_2-O-CH(CH_2OH)_2$ |
| 2-Amino-6-mercapto-9-(1,3-diacetoxypropyl-2-oxymethyl)-purin | " | $-CH_2-O-CH(CH_2OCOCH_3)_2$ |

Die Verbindungen sind z.T. im Handel erhältlich und nach bekannten bzw. Analogie-Verfahren herstellbar. Einige Herstellverfahren sind im nachfolgenden Formelschema dargestellt.

Man kann also z.B.

a) 6- Hydroxypurin oder 2-Amino-6-hydroxypurin (1) mit einem Schwefelungsmittel - vorzugsweise $P_2S_5$ in Dimethylacetamid - zu 6-Mercapto- bzw. 6-Mercapto-2-amino-purin (2), dieses mit einem aliphatischen Säureanhydrid -vorzugsweise mit Acetanhydrid - zur 9-Acyl- bzw. 9-Acyl-2-acylamino-Verbindung (3)

Formelschema für die Herstellung der Verbindungen der Formel I

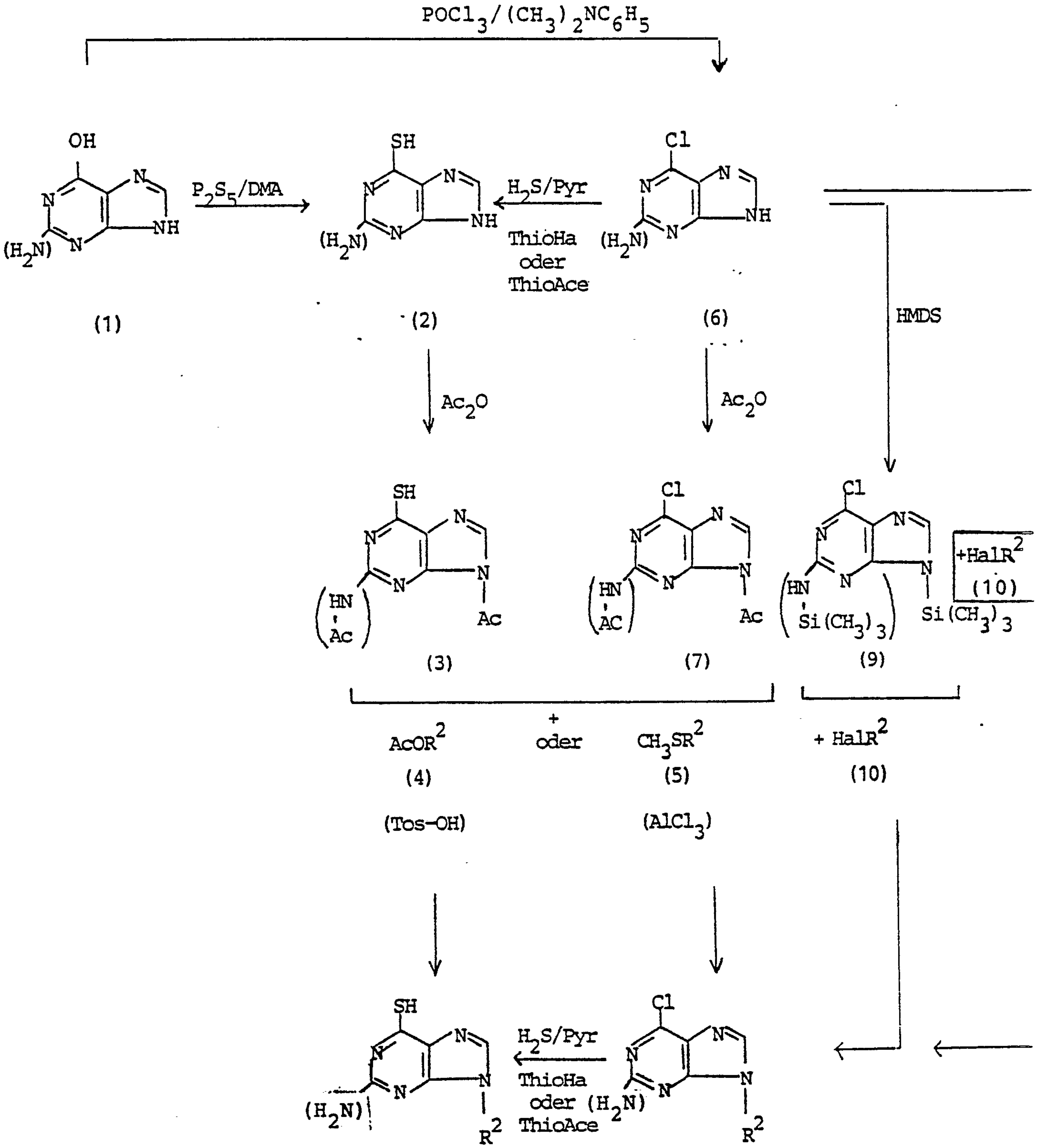


(I) (8)

DMA = Dimethylacetamid
Pyr = Pyridin
ThioHa= Thioharnstoff
ThioAce= Thioacetamid
$Ac_2O$ = Acetanhydrid
TosOH = 4-Toluolsulfonsäure
HMDS = Hexamethyldisilazan $HN[Si(CH_3)_3]_2$

und diese dann mit einer Verbindung der Formel $AcOR^2$ (4); Ac = Acylrest, $R^2$ hat die bei Formel I angegebene Bedeutung) oder $CH_3SR^2$ (5; Bedeutung von $R^2$ wie vorher erwähnt) zur Verbindung I umsetzen, oder

b) wiederum ausgehend von 6-Hydroxy- bzw. 6-Hydroxy-2-amino-purin (1)
dieses mit einem Chlorierungsmittel - vorzugsweise mit $POCl_3/(CH_3)_2NC_6H_5$ - in 6-Chlor- bzw. 6-Chlor-2-amino-purin (6),
dieses mit einem Hydrosulfierungsmittel - vorzugsweise mit $H_2S$/Pyridin, Thioharnstoff oder Thioacetamid - zu 6-Mercapto- bzw. 6-Mercapto-2-amino-purin (2) umsetzen und dann weiter verfahren wie bei a) oder

c) 6-Chlor- bzw. 6-Chlor-2-amino-purin (6; wie z.B. erhalten in der ersten Stufe von b) mit einem aliphatischen Säureanhydrid - vorzugsweise Acetanhydrid - zu 6-Chlor-9-acyl- bzw. 6-Chlor-2-acylamino-9-acyl-purin (7),
dieses mit einer Verbindung der Formel $AcOR^2$ (4) oder $CH_3SR^2$ (5) zu dem in 9-Stellung durch $R^2$ substituierten 6-Chlor- bzw. 6-Chlor-2-amino-purinderivat (8)
und dieses mit einem Hydrosulfierungsmittel zur Verbindung I umsetzen, oder

d) 6-Chlor- bzw. 6-Chlor-2-amino-purin (6) mit Hexamethyldisilazan zu 6-Chlor-9-trimethylsilyl- bzw. 6-Chlor-2-trimethylsilylamino-9-trimethylsilyl-purin (9),
dieses mit Hal-$R^2$ (10; Hal = Halogen, vorzugsweise Cl oder Br, $R^2$ hat die vorerwähnte Bedeutung) zu dem in 9-Stellung durch $R^2$ substituierten 6-Chlor- bzw. 6-Chlor-2-amino-purin (8)
und dieses mit einem Hydrosulfierungsmittel zur Verbindung I umsetzen, oder - für den Fall, daß man nur in der 2-Stellung unsubstituierte 6-Mercaptopurine herstellen will -

e) 6-Chlor-purin (6) direkt mit Hal$R^2$ (10) zu dem in 9-Stellung durch $R^2$ substituierten 6-Chlorpurin (8)
und dieses mit einem Hydrosulfierungsmittel zur Verbindung I umsetzen.

## Ansprüche

1. Verwendung der Verbindungen der nachstehenden Formel I zur Herstellung eines Arzneimittels zur Behandlung von durch Retroviren verursachten Krankheiten:

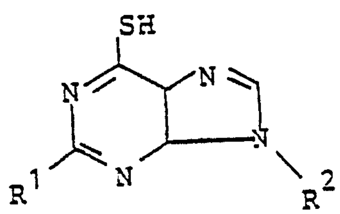

(I)

worin $R^1$ = H, $NH_2$, $NHR^{1'}$ ($R^{1'}$ = subst. Acylrest, vorz. $COCH_3$),

$$R^2 = H,\ -CH_2-X-\underset{R^5}{\underset{|}{C}}H-CH_2-OR^3,$$

X = O oder S
$R^3$ = H, aliphat. $C_1$-$C_6$-Kohlenwasserstoffrest mit ggf. 1 Doppelbindung, $CH_2$-$C_6H_5$, $COR^4$,
$R^4$ = $C_1$-$C_6$-Alkylrest, $CH_2$-$C_6H_5$, $C_6H_5$,
$R^5$ = H, $CH_2$-Y,
Y = H, Halogen, $OR^6$,
$R^6$ = unabhängig von $R^3$ die gleichen Bedeutungen wie $R^3$ mit Ausnahme der Verbindungen, in denen
a) $R^1$ = $NH_2$ und
$R^5$ (in $R^2$) = $CH_2$-Y mit
Y = Halogen, $OR^6$,
$R^6$ = aliphat. $C_1$-$C_6$-Kohlenwasserstoffrest mit ggf. 1 Doppelbindung oder $CH_2$-$C_6H_5$, sowie
b) $R^1$ = H und

$$R^2 = -CH_2-X-\underset{\substack{| \\ R^5}}{CH}-CH_2OR^3 \text{ mit}$$

X = O oder S,
$R^3$ = H, i-$C_3H_7$, sec-$C_4H_9$, $CH_2$-$C_6H_5$, $COR^4$,
$R^4$ = $CH_3$, $C_2H_5$, $C_6H_5$,
$R^5$ = H, $CH_2OC_3H_7$(i), $CH_2OC_4H_9$(sec.), $CH_2OCOR^4$,
wobei die Verbindungen mit gleichzeitig $R^1$ = $R^3$ = $R^5$ = H nicht ausgenommen sind.

2. Verwendung nach Anspruch 1, dadurch gekennzeichnet, daß die Retroviren HIV sind.

3. Verwendung nach Anspruch 1 oder 2, dadurch gekennzeichnet, daß ein Arzneimittel mit einer Dosiseinheit von etwa 30 bis 300 mg, vorzugsweise von etwa 50 bis 250 mg, mindestens einer Verbindung der Formel I hergestellt wird.

4. Verfahren zur Herstellung eines Arzneimittels zur Behandlung von durch Retroviren - insbesondere durch HIV - verursachten Krankheiten, dadurch gekennzeichnet, daß man eine wirksame Menge mindestens einer Verbindung der Formel I (wie in Anspruch 1 definiert) mit üblichen physiologisch verträglichen Zusatzstoffen in eine geeignete Darreichungsform bringt.

5. Verwendung von Verbindungen der Formel I gemäß Anspruch 1 zur Bekämpfung von durch Retroviren verursachten Krankheiten.

6. Verwendung von Verbindungen der Formel I gemäß Anspruch 1 zur Bekämpfung von durch HIV verursachten Krankheiten.